(19) Europäisches Patentamt
European Patent Office
Office européen des brevets

(11) **EP 3 723 585 B1**

(12) **EUROPÄISCHE PATENTSCHRIFT**

(45) Veröffentlichungstag und Bekanntmachung des Hinweises auf die Patenterteilung:
**01.05.2024   Patentblatt 2024/18**

(21) Anmeldenummer: **18815644.2**

(22) Anmeldetag: **06.12.2018**

(51) Internationale Patentklassifikation (IPC):
**A61B 5/263** (2021.01)     H01B 3/08 (2006.01)
H01B 1/02 (2006.01)     H01B 3/02 (2006.01)
A61N 1/05 (2006.01)

(52) Gemeinsame Patentklassifikation (CPC):
**A61B 5/686; A61B 5/263;** A61B 2562/0209;
A61B 2562/028; A61B 2562/222; A61N 1/05;
H01B 1/02; H01B 3/02; H01B 3/08

(86) Internationale Anmeldenummer:
**PCT/EP2018/083737**

(87) Internationale Veröffentlichungsnummer:
**WO 2019/115328 (20.06.2019 Gazette 2019/25)**

(54) **MEHRSCHICHTAUFBAU, SYSTEM, VERWENDUNG UND VERFAHREN**

MULTI-LAYER STRUCTURE, SYSTEM, USE AND METHOD

CONSTRUCTION MULTICOUCHES, SYSTÈME, UTILISATION ET PROCÉDÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.12.2017   DE 102017130152**

(43) Veröffentlichungstag der Anmeldung:
**21.10.2020   Patentblatt 2020/43**

(73) Patentinhaber: **Acquandas GmbH**
**24143 Kiel (DE)**

(72) Erfinder: **LIMA DE MIRANDA, Rodrigo**
**24116 Kiel (DE)**

(74) Vertreter: **Meissner Bolte Partnerschaft mbB**
**Patentanwälte Rechtsanwälte**
**Postfach 86 06 24**
**81633 München (DE)**

(56) Entgegenhaltungen:
WO-A2-2016/132128     US-A1- 2011 003 159
US-A1- 2015 343 749

- **CHRISTOPH BECHTOLD ET AL: "Fabrication of self-expandable NiTi thin film devices with micro-electrode array for bioelectric sensing, stimulation and ablation", BIOMEDICAL MICRODEVICES, Bd. 18, Nr. 6, 10. November 2016 (2016-11-10), XP55573828, NL ISSN: 1387-2176, DOI: 10.1007/s10544-016-0131-6**
- **TILLMANN WOLFGANG ET AL: "Deposition of superelastic composite NiTi based films", VACUUM, PERGAMON PRESS, GB, Bd. 104, 4. Januar 2014 (2014-01-04), Seiten 41-46, XP028662086, ISSN: 0042-207X, DOI: 10.1016/J.VACUUM.2013.12.010**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Processed by Luminess, 75001 PARIS (FR)

**Beschreibung**

Die Erfindung betrifft einen Mehrschichtaufbau, ein System, die Verwendung des Mehrschichtaufbaus und ein Verfahren zur Selbstheilung eines Mehrschichtaufbaus sowie ein Verfahren zum Betrieb eines Mehrschichtaufbaus.

Entsprechende Mehrschichtaufbauten des Stands der Technik werden zum Beispiel beschrieben in CHRISTOPH BECHTOLD ET AL: "Fabrication of self-expandable NiTi thin film devices with micro-electrode array for bioelectric sensing, stimulation and ablation", BIOMEDICAL MICRODEVICES, Bd. 18, Nr. 6 (2016).

Mehrschichtaufbauten werden in der Mikroelektronik, beispielsweise bei Mikroelektrodenarrays (MEA) verwendet. Derartige Elektroden sind dazu vorgesehen, um neuronale Aktivitäten zu überwachen und/oder zu stimulieren. Üblicherweise weisen derartige Mehrschichtaufbauten ein Trägersubstrat auf, auf dem Leiterschichten aufgebracht sind, die vom Trägersubstrat durch elektrisch isolierende Schichten getrennt sind.

Für isoliert-elektrische Verbindungen auf flexiblen Trägersubstraten werden Werkstoffe verwendet, die ähnliche Elastizitäten aufweisen, um eine gewisse Flexibilität des Mehrschichtaufbaus zu erreichen. Dies können anorganisch/polymere Isolationswerkstoffe und metallische Leiterbahnwerkstoffe sein. Bei Verwendung anorganischer/oxidischer Isolationswerkstoffe sind Wechselbelastungen, insbesondere Wechselbelastungen mit größeren Dehnungen schwierig zu realisieren. Im Stand der Technik werden für möglichst dauerhafte, isoliert-elektrische Verbindungen mit einem flexiblen Trägersubstrat polymere/organische Werkstoffen als dünne elektrische Isolatoren verwendet. Polymere neigen zur Alterung und Degeneration im biologischen Milieu, was mittelfristig zu funktionellen Defekten der Isolation und der Leiterbahnen, gerade unter Belastung, führt.

Ein weiterer Ansatz besteht in der Anwendung von alternierenden Multilayer-Dünnschichten (Nanolaminaten) aus organischen/polymeren und anorganischen/oxidischen Substraten. Die Schichthaftung zwischen unterschiedlichen organischen und anorganischen Werkstoffen ist komplex und meist nicht befriedigend. Es kommt zu Isolations-Defekten und zur Degeneration im biologischen Milieu.

Eine Auslegung des Designs und der Isolationsschicht als Mehrfachleiter und -isolatoren ist denkbar, so dass die Schichten unter Belastung keine großen mechanischen Dehnungen erfahren. Dies hat den Nachteil, dass es praktisch unmöglich ist, eine große Dehnung nur durch ein mechanisches Design zu realisieren. Damit werden die Querschnitte kleiner und die möglichen Signalamplituden nehmen ab. Die elektrischen Isolations- und Leitermaterialien können brechen und verlieren damit Ihre elektrischen Funktionseigenschaften bei Verbindungen mit hoch-flexiblen Substrat-Schichten und unter Wechselbelastungen, wenn die Wechselbelastungen relativ niedrige Grenzwerte überschreiten.

Der Erfindung liegt die Aufgabe zu Grunde, einen Mehrschichtaufbau, insbesondere für mikroelektronische Anwendungen, anzugeben, der mechanisch flexibel ist und bei Belastung seine elektrischen Eigenschaften möglichst gut beibehält. Der Erfindung liegt ferner die Aufgabe zu Grunde, ein System mit einem derartigen Mehrschichtaufbau, die Verwendung eines Mehrschichtaufbaus und ein Verfahren zur Selbstheilung eines Mehrschichtaufbaus bzw. zum Betrieb eines Mehrschichtaufbaus anzugeben.

Erfindungsgemäß wird diese Aufgabe mit Blick auf den Mehrschichtaufbau durch den Gegenstand des Anspruchs 1 gelöst. Mit Blick auf das System wird die Aufgabe durch den Gegenstand des Anspruchs 6, mit Blick auf die Verwendung durch den Gegenstand des Anspruchs 7, mit Blick auf das Verfahren zur Selbstheilung durch den Gegenstand des Anspruchs 8 und mit Blick auf das Verfahren zum Betrieb eines Mehrschichtaufbaus durch den Gegenstand des Anspruchs 10 gelöst.

Die Erfindung ermöglicht selbstheilende, isoliert-elektrische Verbindungen, bspw. zur Übertragung oder Detektion elektrischer Signale, Spannungen oder Ströme in z.B. bioelektronischen Implantaten. Die Erfindung geht damit einen anderen Weg als der Stand der Technik. Die Erfindung lässt Risse in der elektrisch isolierenden Schicht an sich zu, da diese wieder geheilt werden. Die Risse werden dabei soweit geschlossen, dass die elektrischen Eigenschaften des Mehrschichtaufbaus insgesamt weniger stark beeinträchtigt werden, als dies im Stand der Technik der Fall ist. Eine vollständige Heilung von Rissen in dem Sinne, dass Risse zumindest makroskopisch vollständig verschwinden, ist nicht zwingend erforderlich. Es genügt, dass die Risse soweit geschlossen werden, dass die ursprünglichen elektrischen Eigenschaften des Mehrschichtaufbaus vor der mechanischen Belastung weitgehend erhalten bleiben.

Für die Selbstheilung ist vorgesehen, dass die Trägerschicht ein Formgedächtnismaterial umfasst, das zur Übertragung von Rückstellkräften auf die elektrisch isolierende Schicht angepasst ist. Die Rückstellkräfte entstehen in an sich bekannter Weise durch die bei Formgedächtnismaterialien inhärente Phasenumwandlung. Für die spannungsinduzierte Phasenumwandlung der Trägerschicht ist erfindungsgemäß vorgesehen, dass diese um mindestens 0,5 % dehnbar ist. Die dabei auftretenden Rückstellkräfte wirken zwischen der Trägerschicht und der elektrisch isolierenden Schicht und führen dazu, dass etwaige, in der elektrisch isolierenden Schicht gebildete Risse geschlossen bzw. weitgehend geschlossen werden. Deshalb werden die zwischen der Trägerschicht und der elektrisch isolierenden Schicht wirkenden Kräfte als Rückstellkräfte bezeichnet, da diese Kräfte die elektrisch isolierende Schicht lokal zumindest weitgehend in den Ausgangszustand bzw. in einen Zustand zurückführen, in dem die Schicht weitgehend rissfrei oder zumindest rissarm ist. Damit kann der erfindungsgemäße Mehrschichtaufbau mit großen Belastungen bzw. Dehnungen beaufschlagt werden, ohne dass die elektrischen Eigenschaften des Mehrschichtaufbaus signifikant beeinträchtigt werden. Insbesondere sind Dehnungen von größer 0,5 % möglich.

Unter Dehnung wird die relative Änderung der Abmessung, insbesondere eine relative Längenänderung (Verlängerung bzw. Verkürzung) der Trägerschicht bzw. allgemein einer Schicht oder des gesamten Mehrschichtaufbaus unter Belastung verstanden. Die Belastung kann beispielsweise durch eine Kraft oder durch eine Temperaturänderung (Wärmeausdehnung) erfolgen. Die die relative Änderung der Abmessung, insbesondere die relative Längenänderung erfolgt hauptsächlich in der von der jeweiligen Schicht aufgespannten Ebene. Wenn die Abmessung des Körpers sich vergrößert, spricht man von einer positiven Dehnung (Streckung), andernfalls von einer negativen Dehnung oder Stauchung.

$$\varepsilon = \frac{\Delta \ell}{\ell_0}$$

Die Dehnung ist definiert als wobei $\Delta l$ die Längenänderung bzw. allgemein die Änderung der Abmessung und $l_0$ die ursprüngliche Länge, bzw. allgemein die ursprüngliche Abmessung ist.

Die Dehnung kann vorzugsweise in einem Bereich von 0,5 % bis 10 % oder mehr betragen. Mit anderen Worten kann die Dehnung 0,5 % bis 10 % betragen. Die Untergrenze für den Bereich der Dehnung beträgt mindestens 0,5 %, vorzugsweise mindestens 1 Prozent, mindestens 1,5 %, mindestens 2 %, mindestens 2,5 %, mindestens 3 %, mindestens 3,5 %, mindestens 4 %.

Die Schichtanordnung der einzelnen Schichten im Mehrschichtaufbau unterliegt keinen besonderen Einschränkungen. Es ist lediglich erforderlich, dass eine Übertragung der Rückstellkräfte von der Trägerschicht auf die elektrisch isolierende Schicht möglich ist. Es ist beispielsweise möglich, dass auf einer einzelnen Trägerschicht mehrere elektrisch isolierende Schichten und elektrisch leitende Schichten abwechselnd angeordnet sind. Ist auch möglich, dass der Mehrschichtaufbau mehrere Schichteinheiten, jeweils umfassend wenigstens eine flexible Trägerschicht, wenigstens eine elektrisch isolierende Schicht und wenigstens eine elektrisch leitende Schicht aufweist. Die Schichteinheiten selbst können wiederum eine einzelne Trägerschicht aufweisen, auf der mehrere elektrisch isolierende Schichten und elektrisch leitende Schichten alternierend angeordnet sind.

Die Erfindung ermöglicht selbstheilende Eigenschaften des Mehrschichtaufbaus im Zusammenhang mit dauerhaften bzw. kontinuierlichen isoliert-elektrischen Verbindungen ebenso wie im Zusammenhang mit diskret isoliert-elektrischen Verbindungen. Elektrische Leiter und Isolatoren mit einer flexiblen Trägerschicht, wie bspw. Nitinol, können unter mechanischen Belastungen oder Wechselbelastungen mit einer Dehnung von größer 0,5% verwendet werden, so dass eine kontinuierliche oder diskrete dauerhafte Übertragung von elektrischen Signalen, Spannungen und Strömen möglich ist.

Bevorzugte Ausführungsformen sind in den Unteransprüchen angegeben.

Wenn die Trägerschicht, die elektrisch isolierende Schicht und die elektrisch leitende Schicht zusammen um mindestens 0,5 % dehnbar sind, wird die Stabilität des Schichtverbundes verbessert. Gleichzeitig wird die für die Phasenumwandlung der Trägerschicht erforderliche Verformung erreicht.

Die Schichtdicke der elektrisch isolierenden Schicht bzw. der elektrisch leitenden Schicht kann jeweils höchstens 50 μm betragen. Andere Schichtdicken sind möglich. Besonders bevorzugt ist eine Schichtdicke der elektrisch isolierenden Schicht zwischen 1 nm und 8 μm.

Vorzugsweise sind die Schichten so dicht aneinander angeordnet, dass Van-der Waals-Kräfte zwischen den Grenzschichten der unterschiedlichen Materialschichten wirken.

Hinsichtlich der Materialien für die Trägerschicht, die elektrisch isolierende Schicht und die elektrisch leitende Schicht wird auf die Ansprüche verwiesen.

Im Rahmen des erfindungsgemäßen Systems wird ein Mehrschichtaufbau nach der Erfindung und ein mechanischer Aktor beansprucht, der mit dem Mehrschichtaufbau zur Bedienung des Mehrschichtaufbaus verbunden ist. Mit anderen Worten ist der mechanische Aktor dazu vorgesehen, die Dehnung des Mehrschichtaufbaus bzw. zumindest der Trägerschicht zu initiieren bzw. zu triggern.

Die Verwendung des erfindungsgemäßen Mehrschichtaufbaus ist nicht auf medizinische Anwendungen beschränkt, wobei diese einen sehr wichtigen Anwendungsfall bilden. Die Erfindung kann in allen möglichen technischen Bereichen eingesetzt werden, bei denen mikroelektronische Bauteile verwendet und Belastungen ausgesetzt werden. Beispiele für entsprechende Verwendungen sind in Anspruch 7 angegeben.

Im Rahmen des erfindungsgemäßen Verfahrens zur Selbstheilung eines Mehrschichtaufbaus nach Anspruch 1 wird dieser um mindestens 0,5 % gedehnt.

Dadurch wird die für die Erzeugung der Rückstellkräfte erforderliche Spannung induziert, die zu der Phasenumwandlung führt.

Wenn die im Betrieb auftretenden Belastung des Mehrschichtaufbaus, beispielsweise eine Wechselbelastung zu einer Dehnung von mindestens 0,5% des Mehrschichtaufbaus, zumindest aber der Trägerschicht, führt, wird eine automatische Selbstheilung etwaiger Risse in der elektrisch isolierenden Schicht erreicht. Die für die Selbstheilung verantwortliche Belastung, insbesondere Wechselbelastung, kann einer anderen im Betrieb auftretenden Belastung überlagert werden, so dass auch dann eine automatische Selbstheilung etwaiger Risse in der elektrisch isolierenden Schicht bewirkt wird.

Im Rahmen des erfindungsgemäßen Verfahrens zum Betrieb eines Mehrschichtaufbaus nach Anspruch 1 wird an die elektrisch leitende Schicht eine elektrische Spannung angelegt. Der Mehrschichtaufbau wird mit einer Wechselbelastung beaufschlagt, bei der der Mehrschichtaufbau um mindestens 0,5% gedehnt wird. Dabei wird die Dehnung so eingestellt wird, dass bei der Wechselbeanspruchung ein kontinuierlicher Strom durch die elektrische Leitung fließt oder dass bei der Wechselbeanspruchung der Strom durch die elektrische Leitung entsprechend der Frequenz der Wechselbeanspruchung unterbrochen wird.

Bei dem erfindungsgemäßen Verfahren zum Betrieb des Mehrschichtaufbaus nach Anspruch 1, beispielsweise als Mikroelektrode, wird permanent oder zumindest während eines längeren zusammenhängenden Zeitraumes eine Wechselbelastung auf den Mehrschichtaufbau aufgeprägt. Die Wechselbelastung führt dazu, dass die Rückstellkräfte zwischen der Trägerschicht und der elektrisch isolierenden Schicht permanent bzw. während eines längeren Zeitraumes wirken, sodass eine kontinuierliche selbstheilende Wirkung erzeugt wird.

Dabei sind zwei unterschiedliche Betriebsmöglichkeiten bzw. Betriebszustände zu unterscheiden. Die im Zusammenhang mit der Wechselbelastung erzeugte Dehnung kann so niedrig sein (allerdings nicht niedriger als 0,5 %), dass ein kontinuierlicher, unterbrechungsfreier Strom durch die elektrisch leitende Schicht fließt. Alternativ kann die Wechselbelastung so hoch eingestellt sein, dass in einem maximalen Amplitudenbereich der Wechselbelastung eine Unterbrechung des Stromflusses durch die elektrisch leitende Schicht erfolgt, sodass der Strom diskret, d.h. nicht-kontinuierlich durch die elektrisch leitende Schicht fließt.

Die Erfindung wird nachstehend anhand von Ausführungsbeispielen und unter Bezug auf die beigefügten schematischen Zeichnungen mit weiteren Einzelheiten näher beschrieben.

In diesen zeigen

Fig. 1     einen Querschnitt durch einen Mehrschichtaufbau mit einer Trägerschicht, einer elektrisch isolierenden Schicht und einer elektrisch leitenden Schicht nach einem erfindungsgemäßen Ausführungsbeispiel;

Fig. 2     einen Querschnitt durch einen Mehrschichtaufbau nach einem erfindungsgemäßen Ausführungsbeispiel vor Aufbringen einer Belastung, während der Belastung und nach der Belastung; und

Fig. 3     ein Diagramm, das den Verlauf des Widerstandes in Abhängigkeit von einer Wechselbelastung über der Zeit darstellt.

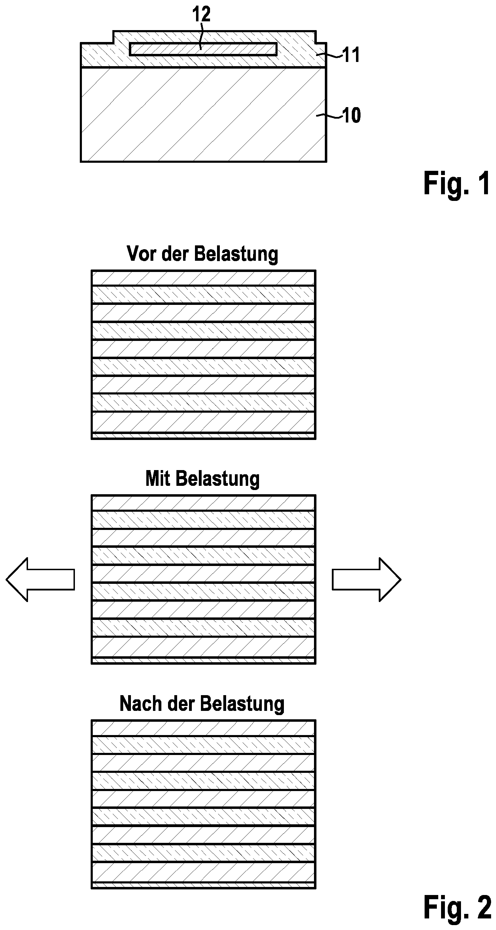

Fig. 1 zeigt einen Querschnitt durch einen Mehrschichtaufbau nach einem erfindungsgemäßen Ausführungsbeispiel. Dabei kann es sich beispielsweise um eine flexible, elektrisch-isolierte Verbindung handeln, die allgemein als Mehrschichtdevice oder als Mehrschichtsystem bezeichnet werden kann. Der Mehrschichtaufbau bildet eine zentrale Komponente des Mehrschichtsystems. Ein Beispiel für ein Mehrschichtsystem ist ein Mehrkanalverbinder (multichannel connector). Der dargestellte Mehrschichtaufbau wird bevorzugt im medizintechnischen Bereich eingesetzt. Andere Anwendungen sind möglich.

Beispiele für derartige Anwendungen sind Anwendungen

- in einem medizinischen, bioelektronischen Implantat, insbesondere zur elektrischen Detektion und Stimulation von biologischem Gewebe,
- in einem Sensor oder BioMEMS als elektrisch isolierte Leiterbahn,
- zur Detektion biologischer Signale,
- in medizinischen, industriellen und Lifestyle Anwendungen als elektrisch isolierte Leiterbahn zur Übertragung elektrischer Signale, Spannungen oder Ströme,
- in Verbindungssteckern und Verbindungskonnektoren als elektrisch isolierte Verbindung,
- in Verbindungen zu Implantaten und Wearables als elektrisch isolierte Verbindung.

Der Mehrschichtaufbau gemäß Fig. 1 ist dreilagig aufgebaut. Auf einer Trägerschicht 10 ist eine elektrisch isolierende Schicht 11 aufgebracht. Auf der elektrisch isolierenden Schicht 11 ist eine elektrisch leitende Schicht 12 aufgebracht. Die elektrisch leitende Schicht 12 ist durch die elektrisch isolierende Schicht 11 von der Trägerschicht 10 elektrisch isoliert. Bei dem Beispiel gemäß Fig. 1 ist die elektrisch leitende Schicht 12 von der elektrisch isolierenden Schicht 11 ummantelt, sodass sowohl die der Trägerschicht 10 zugewandte Seite als auch die von der Trägerschicht 10 abgewandte Seite der elektrisch leitenden Schicht 12 elektrisch isoliert sind.

Der Mehrschichtaufbau kann mehrere elektrisch isolierende Schichten 11 und elektrisch leitende Schichten 12 in Sandwichbauweise bzw. abwechselnd übereinander aufweisen. Die elektrisch leitende Schicht 12 bildet Leiterbahnen, die zur Funktion des Mehrschichtaufbaus bzw. des entsprechenden Systems verschaltet sind.

Die Trägerschicht 10 ist aus einem Formgedächtniswerkstoff hergestellt. Im Beispiel gemäß Fig. 1 ist dazu eine Nickel-Titanlegierung verwendet. Andere Formgedächtniswerkstoffe sind möglich.

Das Material der Trägerschicht ist aus der Gruppe

- NiTi-Legierungen,

- Polymere

ausgewählt.

Die Trägerschicht ist um mindestens 0,5 % dehnbar. Konkret ist der gesamte Mehrschichtaufbau um 0,5 % dehnbar. Durch eine entsprechende Dehnung wird eine spannungsindizierte Phasenumwandlung in der Trägerschicht bewirkt, so dass entsprechende Kräfte, d.h. Rückstellkräfte, von der Trägerschicht 10 auf die elektrisch isolierende Schicht 11 übertragen werden. Etwaige in der elektrisch isolierenden Schicht 11 gebildete Risse werden durch diese Kräfte eliminiert bzw. geheilt. Eine vollständige Eliminierung ist nicht erforderlich. Es genügt, wenn in die elektrisch isolierende Schicht 11 nach der Belastung rissärmer ist als vor der Belastung.

Im optimalen Fall ist die elektrisch isolierende Schicht 11 vor der Belastung rissfrei. Während und nach der Belastung werden etwaige Risse durch die von der Trägerschicht 10 erzeugten Kräfte unterdrückt bzw. geheilt.

Die Trägerschicht 10 ist flexibel.

Wie aus Fig. 1 hervorgeht, ist die Schichtdicke der Trägerschicht 10 größer als die Schichtdicke der elektrisch isolierenden Schicht 11 und der elektrisch leitenden Schicht 12 zusammen. Andere Verhältnisse sind möglich. Beispielsweise beträgt die Schichtdicke der elektrisch isolierenden Schicht 11 600 nm, d.h. die Schichtdicke zwischen der elektrisch leitenden Schicht 12 und der Trägerschicht 10 beträgt 600 nm. Die Schichtdicke der elektrisch leitenden Schicht beträgt bei diesem Ausführungsbeispiel 300 nm. Die Schichtdicke des Isolators auf der Oberseite bzw. auf der von der Trägerschicht 10 abgewandten Seite der elektrisch leitenden Schicht 11 beträgt bei dem Ausführungsbeispiel 300 nm. Die Schichtdicke der Trägerschicht 10 kann beispielsweise 30 $\mu$m betragen. Andere Schichtdicken sind möglich.

Generell kann die Schichtdicke der elektrisch isolierenden Schicht 11 zwischen 1 nm und 8 $\mu$m betragen.

Zu den Materialien der elektrisch isolierenden Schicht 11 und der elektrisch leitenden Schicht 12 wird auf die Ansprüche 1 und 4 verwiesen.

In Fig. 2 ist ein Querschnitt durch einen Mehrschichtaufbau nach einem erfindungsgemäßen Beispiel gezeigt. Die obere Darstellung in Figur 2 zeigt einen Querschnitt durch die einzelnen Schichten vor deren Belastung. Die mittlere Darstellung zeigt die einzelnen Schichten während der Belastung. Die untere Darstellung zeigt die einzelnen Schichten nach der Belastung. Im Ergebnis entsprechen die Schichten während und nach der Belastung im Wesentlichen den rissfreien Schichten vor der Belastung. Es ist praktisch kein Unterschied zu erkennen. Dies ist auf den Selbstheilungseffekt des Mehrschichtaufbaus nach dem erfindungsgemäßen Beispiel zurückzuführen.

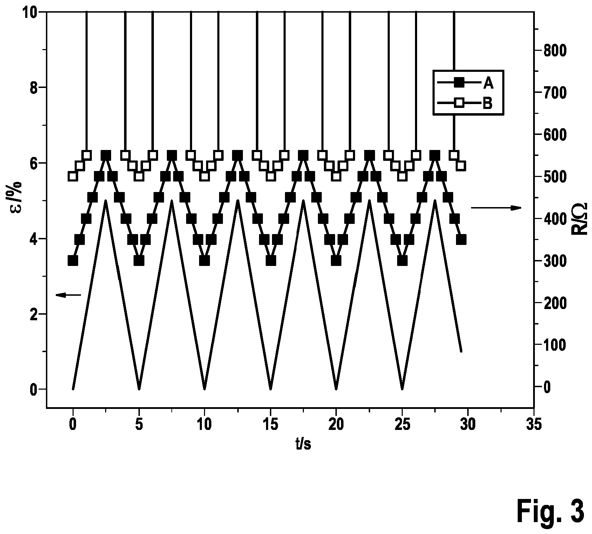

Fig. 3 zeigt anhand eines Diagramms zwei verschiedene Verfahren zum Betrieb eines Mehrschichtaufbaus nach einem erfindungsgemäßen Beispiel, beispielsweise im Rahmen einer der obigen Verwendungen. Das Verfahren beruht darauf, dass der Mehrschichtaufbau mit einer Wechselbelastung beaufschlagt wird, so dass es zu einem kontinuierlichen Selbstheilungseffekt kommt, wie oben beschrieben.

Bei dem Verfahren A besteht eine dauerhafte und kontinuierliche elektrische Verbindung. Die elektrische Leiterbahn auf dem Isolator verändert den elektrischen Widerstand unter Wechselbelastung. Mit zunehmender Dehnung steigt der Widerstand an, mit abnehmender Dehnung des Trägersubstrates nimmt der Widerstand ab. Die Isolation und elektrische Leitung sind aber kontinuierlich vorhanden und dauerbelastbar.

Bei dem Verfahren B kommt es zu einer diskreten elektrischen Verbindung. Die elektrische Verbindung ist periodisch unterbrochen und zwar mit der Frequenz der Wechselbelastung. Mit dem Überschreiten eines kritischen Dehnungswertes kommt es zu einem Abriss der Verbindung. Fällt die Dehnung unter diesen kritischen Wert, ist die elektrische Verbindung wieder vorhanden und kontinuierlich. Diese Prozesse sind praktisch unendlich reproduzierbar.

Mögliche Herstellungsverfahren sind:

- Physikalische Gasphasenabscheidung (PVD), unter anderem Magnetron-Sputterabscheidung

- Chemische Gasphasenabscheidung (CVD), u.a. Atomic Layer Deposition, PECVD,

- Thermische Abscheidung

Eine Formgebung des Mehrschichtaufbaus durch thermomechanische Wärmebehandlung ist möglich. Dies kann bspw. durch Kristallisation des amorph abgeschiedenen Formgedächtnismaterials unter mechanischer Belastung durch eine Wärmebehandlung im Hochvakuumofen erfolgen.

Der Schutzumfang der Erfindung ist in den anhängenden Ansprüchen definiert.

**Patentansprüche**

1. Mehrschichtaufbau mit

   - wenigstens einer flexiblen Trägerschicht (10),
   - wenigstens einer elektrisch isolierenden Schicht (11), und
   - wenigstens einer elektrisch leitenden Schicht (12), wobei die elektrisch isolierende Schicht (11) zwischen der Trägerschicht (10) und der elektrisch leitenden Schicht (12) angeordnet und mit diesen jeweils verbunden ist, wobei zumindest die Trägerschicht (10) um mindestens 0,5% dehnbar ist und ein Formgedächtnismaterial umfasst, das zur Übertragung von Rückstellkräften zur Heilung von Rissen in der elektrisch isolierenden Schicht (11) angepasst ist, indem das Material der Trägerschicht (10) aus der Gruppe

- NiTi-Legierungen, und
- Polymere ausgewählt ist,

**dadurch gekennzeichnet, dass** das Material der elektrisch isolierenden Schicht (11) ZrO2 (stabilisiert mit (Y, Ca, Mg, Ce, Al, Hf)-Oxiden) ist,
wobei
Zusätze aus der Gruppe

- Y2O3
- WO2
- MoO3, MoO2
- ZnO
- MgO
- CaO
- Na2O
- P2O5
- Fe2O3

ausgewählt sind.

2. Mehrschichtaufbau nach Anspruch 1,
**dadurch gekennzeichnet, dass**
die Trägerschicht (10), die elektrisch isolierende Schicht (11) und die elektrisch leitende Schicht (12) zusammen um mindestens 0,5% dehnbar sind.

3. Mehrschichtaufbau nach Anspruch 1 oder 2,
**dadurch gekennzeichnet, dass**
Van-der Waals-Kräfte zwischen den Grenzschichten der unterschiedlichen Materialschichten wirken.

4. Mehrschichtaufbau nach einem der vorhergehenden Ansprüche,
**dadurch gekennzeichnet, dass**
das Material der elektrisch leitenden Schicht (12) aus der Gruppe

- NiTi-Legierungen,
- PtIr-Legierungen,
- Ta und Legierungen davon,
- Pt und Legierungen davon,
- Au und Legierungen davon,
- Ag und Legierungen davon,
- polymere Materialien und
- kohlenstoffhaltige Materialien

ausgewählt ist.

5. Mehrschichtaufbau nach einem der vorhergehenden Ansprüche,
**dadurch gekennzeichnet, dass**
die Schichtdicke der elektrisch isolierenden Schicht (11) zwischen 1 nm und 8 μm beträgt.

6. System mit einem Mehrschichtaufbau nach Anspruch 1 und einem mechanischen Aktor, der mit dem Mehrschichtaufbau zur Dehnung des Mehrschichtaufbaus verbunden ist.

7. Verwendung des Mehrschichtaufbaus nach Anspruch 1,

- in einem medizinischen, bioelektronischen Implantat, insbesondere zur elektrischen Detektion und Stimulation von biologischem Gewebe,
- in einem Sensor oder BioMEMS als elektrisch isolierte Leiterbahn,
- zur Detektion biologischer Signale,
- in medizinischen, industriellen und Lifestyle Anwendungen als elektrisch isolierte Leiterbahn zur Übertragung elektrischer Signale, Spannungen oder Ströme,
- in Verbindungssteckern und Verbindungskonnektoren als elektrisch isolierte Verbindung,
- in Verbindungen zu Implantaten und Wearables als elektrisch isolierte Verbindung.

8. Verfahren zur Selbstheilung eines Mehrschichtaufbaus nach Anspruch 1, bei dem der Mehrschichtaufbau um mindestens 0,5% gedehnt wird.

9. Verfahren nach Anspruch 8,
**dadurch gekennzeichnet, dass**
der Mehrschichtaufbau zur Dehnung mit einer Wechselbelastung beaufschlagt wird.

10. Verfahren nach Anspruch 8 zum Betrieb eines Mehrschichtaufbaus nach Anspruch 1, be dem an die elektrisch leitende Schicht eine elektrische Spannung angelegt wird und der Mehrschichtaufbau mit einer Wechselbelastung beaufschlagt wird, bei der der Mehrschichtaufbau um mindestens 0,5% gedehnt wird, wobei die Dehnung so eingestellt wird, dass bei der Wechselbeanspruchung ein kontinuierlicher Strom durch die elektrische Leitung fließt oder dass bei der Wechselbeanspruchung der Strom durch die elektrische Leitung entsprechend der Frequenz der Wechselbeanspruchung unterbrochen wird.

11. Verfahren nach einem der Ansprüche 9 - 10
**dadurch gekennzeichnet, dass**
die Trägerschicht nach der Belastung eine Restdehnung von höchstens 1% aufweist.

## Claims

1. A multi-layer structure with

- at least one flexible carrier layer (10),
- at least one electrically insulating layer (11) and
- at least one electrically conductive layer (12),
wherein the electrically insulating layer (11) is arranged between the carrier layer (10) and the electrically conductive layer (12) and is connected to each of these, wherein at least the carrier layer (10) is stretchable by at least 0.5% and comprises a shape memory material adapted to transmit restoring forces to heal cracks in the electrically insulating layer (11), in that the material of the carrier layer (10) is selected from the group
- NiTi alloys and
- polymers,

    **characterized in that** the material of the electrically insulating layer (11) is $ZrO_2$ (stabilized with (Y, Ca, Mg, Ce, Al, Hf) oxides), wherein additives are selected from the group

- $Y_2O_3$
- $WO_2$
- $MoO_3$, $MoO_2$
- ZnO
- MgO
- CaO
- $Na_2O$
- $P_2O_5$
- $Fe_2O_2$.

2. The multi-layer structure according to claim 1, **characterized in that**
the carrier layer (10), the electrically insulating layer (11) and the electrically conductive layer (12) together are stretchable by at least 0.5%.

3. The multi-layer structure according to claim 1 or 2, **characterized in that**
van der Waals forces act between the interface layers of the different material layers.

4. The multi-layer structure according to any one of the preceding claims, **characterized in that**
the material of the electrically conductive layer (12) is selected from the group

    - NiTi alloys,
    - PtIr alloys,
    - Ta and alloys thereof,
    - Pt and alloys thereof,
    - Au and alloys thereof,
    - Ag and alloys thereof,
    - polymer materials and
    - carbon-containing materials.

5. The multi-layer structure according to any one of the preceding claims, **characterized in that**
the layer thickness of the electrically insulating layer (11) is between 1 nm and 8 $\mu$m.

6. A system with a multi-layer structure according to claim 1 and a mechanical actuator that is connected to the multi-layer structure for stretching the multi-layer structure.

7. Use of the multi-layer structure according to claim 1,

    - in a medical, bio-electronic implant, particularly for the electric detection and stimulation of biological tissue,
    - in a sensor or BioMEMS as an electrically insulated conductor,
    - for detecting biological signals,
    - in medical, industrial, and lifestyle applications as an electrically insulated conductor for transmitting electrical signals, voltages or currents,
    - in connection plugs and connectors as electrically insulated connections,
    - in connections to implants and wearables as an electrically insulated connection.

8. A method for the self-healing of a multi-layer structure according to claim 1, in which the multi-layer structure is stretched by at least 0.5%.

9. The method according to claim 8, **characterized in that**
the multi-layer structure is subjected to a cyclic load for stretching.

10. The method according to claim 8 for operating a multi-layer structure according to claim 1, in which an electrical voltage is applied to the electrically conductive layer, in which the multi-layer structure is subjected to a cyclic load, in which the multi-layer structure is stretched by at least 0.5%, wherein the deformation is adjusted such that during the cyclic loading a continuous current flows through the electrical line or that during the cyclic loading the current through the electrical line is interrupted according to the frequency of the cyclic loading.

11. The method according to any one of claims 9 - 10, **characterized in that**
the carrier layer has a residual strain of at most 1% after loading.

**Revendications**

1. Structure multicouche, comportant

    - au moins une couche de support (10) flexible,
    - au moins une couche électriquement isolante (11), et
    - au moins une couche électriquement conductrice (12),

la couche électriquement isolante (11) étant disposée entre la couche de support (10) et la couche électriquement conductrice (12) tout en étant reliée à chacune d'elles, au moins la couche de support (10) pouvant subir une élongation d'au moins 0,5 % et comprenant un matériau à mémoire de forme qui est adapté à transmettre des forces de rappel permettant un traitement réparateur de fentes dans la couche électriquement isolante (11), en choisissant le matériau de la couche de support (10) dans le groupe
- des alliages NiTi et
- des polymères,

   **caractérisée en ce que** le matériau de la couche électriquement isolante (11) est du $ZrO_2$ (stabilisé avec des oxydes de (Y, Ca, Mg, Ce, Al, Hf),
   des additifs étant choisis dans le groupe

- $Y_2O_3$
- $WO_2$
- $MoO_3$, $MoO_2$
- $ZnO$
- $MgO$
- $CaO$
- $Na_2O$
- $P_2O_5$
- $Fe_2O_3$.

2. Structure multicouche selon la revendication 1, **caractérisée en ce que**
   la couche de support (10), la couche électriquement isolante (11) et la couche électriquement conductrice (12) pouvant, prises dans leur ensemble, subir une élongation d'au moins 0,5 %.

3. Structure multicouche selon les revendications 1 ou 2, **caractérisée en ce que**
   des forces de Van-der-Waals agissent entre les couches limites des différentes couches de matériaux.

4. Structure multicouche selon l'une des revendications précédentes,
   **caractérisée en ce que**
   le matériau de la couche électriquement conductrice (12) est choisi dans le groupe constitué par

   - les alliages NiTi,
   - les alliages PtIr,
   - le Ta et ses alliages,
   - le Pt et ses alliages,
   - le Au et ses alliages,
   - le Ag et ses alliages,
   - les matériaux polymères, et
   - les matériaux contenant du carbone.

5. Structure multicouche selon l'une des revendications précédentes,
   **caractérisée en ce que**
   l'épaisseur de couche de la couche électriquement isolante (11) est comprise entre 1 nm et 8 µm.

6. Système comportant une structure multicouche selon la revendication 1 et un actionneur mécanique qui est relié à ladite structure multicouche pour effectuer l'élongation de la structure multicouche.

7. Utilisation de la structure multicouche selon la revendication 1,

   - dans un implant bioélectronique médical, destiné notamment à la détection électrique et la stimulation de tissus biologiques,
   - dans un capteur ou BioMEMS en tant que tracé conducteur électriquement isolé,
   - pour détecter des signaux biologiques,
   - dans des applications médicales, industrielles et Lifestyle en tant que tracé conducteur électriquement isolé permettant de transmettre des signaux, tensions et courants électriques,
   - dans des fiches de connexion et les connecteurs en tant qu'élément de connexion électriquement isolé,
   - dans des connexions vers les implants et les équipements portables en tant qu'élément de connexion électriquement isolé.

8. Procédé d'auto-réparation d'une structure multicouche selon la revendication 1, dans lequel ladite structure multicouche subit une élongation d'au moins 0,5 %.

9. Procédé selon la revendication 8,
   **caractérisé en ce que**
   la structure multicouche est soumise à une charge alternée pour effectuer l'élongation.

10. Procédé selon la revendication 8, destiné à mettre en œuvre une structure multicouche selon la revendication 1, consistant à appliquer une tension électrique à la couche électriquement conductrice et à soumettre la structure multicouche à une charge alternée, pour ainsi provoquer une élongation d'au moins 0,5 % de la structure multicouche, ladite élongation étant réglée de manière à ce que, lors de l'application de la charge alternée, un courant circule de manière ininterrompue dans la ligne électrique ou que, lors de l'application de la chargé alternée, le courant circulant dans la ligne électrique soit interrompu selon la fréquence de la charge alternée.

11. Procédé selon l'une des revendications 9 à 10, **caractérisé en ce que**
    la couche de support présente, après l'application

**EP 3 723 585 B1**

de la charge, une élongation résiduelle inférieure ou égale à 1 %.

Fig. 1

Vor der Belastung

Mit Belastung

Nach der Belastung

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **CHRISTOPH BECHTOLD et al.** Fabrication of self-expandable NiTi thin film devices with micro-electrode array for bioelectric sensing, stimulation and ablation. *BIOMEDICAL MICRODEVICES,* 2016, vol. 18 (6